# EUROPEAN PATENT APPLICATION

(11) **EP 4 162 893 A1**
(43) Date of publication of application: **12.04.2023**
(21) Application number: 21306401.7
(22) Date of filing: 06.10.2021
(51) Int. Cl.: A61B 34/10, A61B 90/00, A61B 34/30, A61B 34/32, A61B 34/37, A61B 34/20

(54) **METHOD FOR DEFINING A PROHIBITED VOLUME FOR A SURGICAL ROBOTIC SYSTEM**

(71) Applicant: Ecential Robotics, 38610 Gieres (FR)
(72) Inventor: DAUNE, Gautier, 38610 GIERES (FR); PIERRE, Arnaud, 38700 LA TRONCHE (FR)
(74) Representative: Regimbeau

(57) **Abstract**

The invention relates to a method for determining a prohibited volume for a surgical robotic system comprising a control unit, a robotic arm (102) and an end-effector (105) adapted to treat a region of interest of a patient's body, comprising:
- implementing a first path of a motorized C-arm (403) supporting an X-ray source (401), an X-ray image detector (402) and an anti-collision device (404), said first path comprising at least two different angular positions around a rotation axis of the C-arm;
- at each angular position of the C-arm along said first path, detecting with the anti-collision device an external surface of the patient's body and recording volumetric data of a patient's body portion enclosing the region of interest;
- computing a volumetric model of the patient's body portion based on said volumetric data;
- sending the volumetric model to the control unit of the surgical robotic system,
- based on said volumetric model, defining a prohibited volume (V1, V2, V3) for the robotic arm and end-effector.

## Description

### TECHNICAL FIELD

The present disclosure relates to a method for defining a prohibited volume for a surgical robotic system.

### TECHNICAL BACKGROUND

There is an increasing use of surgical robotic systems for assisting a user (e.g. a surgeon) during a surgical intervention.

For example, in spine surgery, the user may have to implant one or several screws into at least one vertebra. A robotic arm may assist the user by holding a drill guide and maintaining the drill guide according to a planned axis. The user may thus use a handheld drill passing through the drill guide held by the robotic arm to drill a hole intended to receive the screw in a vertebra along the planned axis.

In this regard, the use of a localization system that can localize trackers in real-time (high frequency, low latency) may be used to carry out such manipulation, either in assistance of the surgeon or autonomously. Both the anatomical structure and the surgical tool can be localized, which allows determining in real time the relative positions of the surgical tool relative to the anatomical structure to be treated.

To that end, both the surgical tool and the anatomical structure may comprise a tracker rigidly attached thereto, each tracker being tracked by the localization system.

In some circumstances, a tracker may be rigidly attached to a part of the robotic system and not the surgical tool directly, allowing indirect localization of the tool based on knowledge at any time of the kinematic model of the robotic system between the tracker and the tool.

However, an autonomous manipulation by a robotic system of a surgical tool for treating an anatomical structure comes with important safety issues. Indeed, after the planning of the trajectory of the surgical tool, the surgeon does not act in a direct manner to execute the planned surgical procedure. Moreover, the tracker attached to the anatomical structure may move, e.g. as a result of an involuntary shock or push applied to it. Or the tracker attached to the surgical tool can be off its calibration. Or any software error can lead to a geometric error leading to a false position of the autonomous robot while it executes its planned trajectory. In a similar fashion, a tracker could be defective for numerous reasons (blood spilled on it in case of optical tracking, electromagnetic disturbances in case of electromagnetic tracking), resulting in erroneous tracking.

### SUMMARY OF THE DISCLOSURE

A goal of the present disclosure is to enhance the safety of robotically-assisted or robotically-conducted surgical procedures.

To that end, the present disclosure proposes a method for defining a prohibited volume for a robotic surgical system comprising a control unit, a robotic arm and an end-effector adapted to treat a region of interest of a patient's body, comprising:
- implementing a first path of a motorized C-arm supporting an X-ray source, an X-ray image detector and an anti-collision device, said first path comprising at least two different angular positions around a rotation axis of the C-arm;
- at each angular position of the C-arm along said first path, detecting with the anti-collision device an external surface of the patient's body and recording volumetric data of a patient's body portion enclosing the region of interest;
- computing a volumetric model of the patient's body portion based on said volumetric data;
- sending the volumetric model to the control unit of the surgical robotic system,
- based on said volumetric model, defining a prohibited volume for the robotic arm and end-effector.

By "prohibited volume" is meant in the present text a volume in the coordinate system of the surgical robotic system characterized by the fact that the surgical robotic system is configured to never enter in this volume. More precisely, it means that any part of the surgical robotic system (robotic arm, end-effector, and even the instrument coupled to the end-effector if its dimensions are known by the surgical robotic system) is forbidden to enter in said volume.

The prohibited volume is chosen to enclose at least part of the patient's body, in order to reduce risks of injury of the patient by the robotic arm, in particular during displacement of the robotic arm to guide the surgical tool according to a planned trajectory.

In some embodiments, the prohibited volume is identical to the volume of the volumetric model of the patient's body portion.

In other embodiments, the prohibited volume is computed to enclose the volumetric model of the patient's body portion.

In particular, the prohibited volume may be computed to include a safety zone extending outside the volumetric model of the patient's body portion.

In some embodiments, the prohibited volume comprises first and second sub-volumes each enclosing the volumetric model of the patient's body portion, the first sub-volume being enclosed in the second sub-volume.

In some embodiments, the prohibited volume comprises a first sub-volume enclosing a region of the volumetric model and at least one second sub-volume enclosing the first sub-volume.

The method may further comprise receiving at least one planned target axis or plane, computing an authorized volume around said planned target axis or plane and excluding said authorized volume from the prohibited volume.

The method may also advantageously include generating a warning signal if the robotic arm and/or the end effector moves toward the prohibited volume.

Such a warning signal may also be generated if the robotic arm and/or the end effector is at a distance from the prohibited volume smaller than a predetermined distance.

The anti-collision device may comprise at least one of: a proximity sensor, a telemeter, a LIDAR, a stereo camera, an ultrasound detector, a time-of-flight camera and a tactile sensor.

### BRIEF DESCRIPTION OF THE FIGURES

Further features and advantages will appear in the following description, based on appended drawings, in which:
- FIG. 1 is a general overview of a surgical site including an operating table, a motorized C-arm and a surgical robotic system;
- FIG. 2 schematically illustrates a motorized C-arm, an operating table and a patient's body;
- FIG. 3 is an enlarged view of the X-ray detector and the anti-collision system;
- FIGS. 4A and 4B schematically illustrate two different positions of the X-ray detector relative to the patient's body along a path of the motorized C-arm;
- FIG. 5 illustrates a plurality of positions of the X-ray detector with respect to the patient's body along a path of the motorized C-arm (top) and a graph presenting the distance dₙ between the X-ray detector and the outside surface of the patient's body at each respective position n (bottom);
- FIGS. 6A and 6B schematically illustrate side and top views of a first embodiment of the prohibited volume;
- FIGS. 7A and 7B schematically illustrate side and top views of a second embodiment of the prohibited volume;
- FIGS. 8A and 8B schematically illustrate side and top views of a third embodiment of the prohibited volume;
- FIGS. 9A and 9B schematically illustrate side and top views of a fourth embodiment of the prohibited volume;
- FIGS. 10A and 10B schematically illustrate side and top views of a fifth embodiment of the prohibited volume;
- FIG. 11 is a flow chart illustrating the workflow of a unified control system allowing implementation of the method.

For the sake of legibility, the figures may have not been drawn to scale. Reference signs identical from one figure to another one represent a same element, which may not be described in detail again.

### DETAILED DESCRIPTION OF EMBODIMENTS

The method may be implemented for operating a robotic system in the context of a surgical intervention carried out onto a patient's bone, including but not limited to: implantation of orthopaedic implants such as pedicular screws in the spine, implantation of various orthopaedic implants in bones, reduction and fixation of fractures during traumatological procedures, positioning guides or canulae at a desired position with respect to a predefined target, or insertion of catheters or stents during cardio-vascular or urology procedures.

In this regard, the surgical robotic system is coupled to an X-ray imaging system and may also be coupled to a localization system.

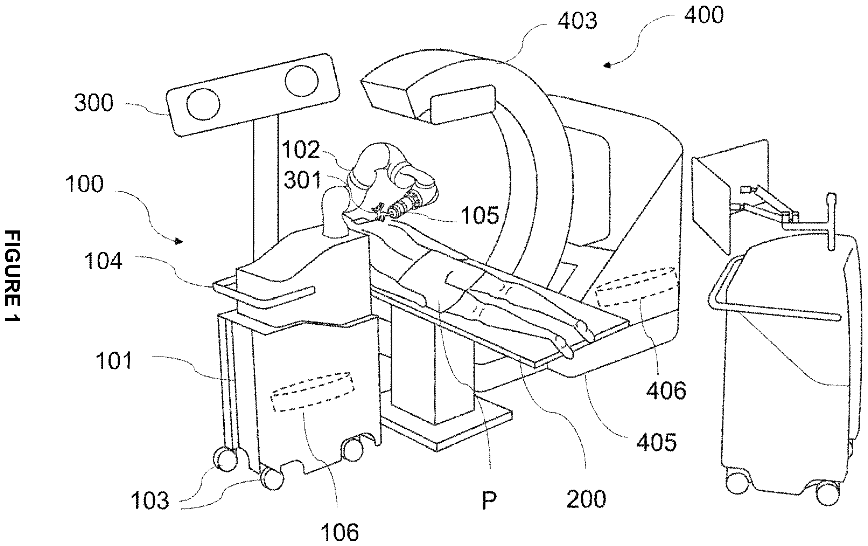

FIG. 1 is a general overview of a surgical site including a surgical robotic system 100, an operating table 200, a localization system 300 and a motorized C-arm 400.

### Surgical robotic system

The surgical robotic system 100 has a movable cart 101 and a robotic arm 102 carried by the movable cart.

During a surgical operation, the cart 101 is intended to remain fixed relative to an operating table 200 on which a patient P lies, while the robotic arm 102 is moved to reach the surgical target(s).

The movable cart 101 may be mobile on wheels 103 and include at least one handle 104 to allow an operator to easily maneuver and transport the surgical robotic system.

The movable cart 101 may be manually actuated or, alternatively, motorized with at least one degree of freedom. At least one of the wheels may be blocked once the movable cart has been moved to the desired position with respect to the operating table.

The robotic arm 102 comprises a plurality of degrees of freedom in translation and/or rotation. Usually, the robotic arm comprises at least six motorized degrees of freedom. To that end, the robotic arm comprises a plurality of articulated segments driven by motors with encoders. By convention, the segments are numbered from the proximal end (which is the end closest to the movable cart) to the distal end (opposite the proximal end) of the robotic arm. The successive robotic arm joints may be rotations or translations. Successive rotations may be orthogonal or parallel. Some parts of the robotic arm may also use parallel mechanisms such as a hexapod architecture.

In preferred embodiments, the robotic arm is made of six or seven rotation axes arranged in an anthropomorphic architecture, such as for example the KUKA liwa^{™} or LBR

Med^{™}, STAUBLI Puma 200^{™}, or KINOVA Gen 3^{™}. In such anthropomorphic architectures, the first and second axes are substantially orthogonal to each other.

The robotic arm may be either controlled in an autonomous mode according to desired targets and trajectories, or manipulated using a collaborative mode (cobot), or telemanipulated using a master control device. A combination of two or more of these different modes may be used on the same robotic system.

The robotic system comprises an end effector 105 for holding a medical device such as a surgical tool or a surgical tool guide, said end effector being mechanically coupled to the distal end of the robotic arm 102.

The surgical robotic system 100 also includes a control unit 106 configured to controllably move the robotic arm 102. In some embodiments, the control unit is configured to also controllably move at least part of the movable cart 102 (e.g. at least one wheel).

The control unit 106 comprises a processor, a data storage device and a communication device. The control unit may advantageously be embedded in the movable cart 101. The movable cart may also comprise switches, such as power switches, an emergency button or the like.

In other embodiments (not illustrated), the control unit may be provided separate from the movable cart, and may be configured to communicate wirelessly or by wires with the robotic arm.

### Localization system

The localization system is configured to localize trackers attached to the patient, the robotic arm, the end-effector and/or the surgical tool or guide. The localization system may be chosen among various technologies, such as optical localization, electromagnetic localization, or ultrasound localization.

In the embodiment illustrated in FIG. 1, the localization system comprises a camera 300 arranged to detect optical trackers, wherein each optical tracker comprises a set of reflective markers, having for example a spherical shape. Only one tracker 301 has been represented attached to the end-effector 105, but at least one additional tracker attached to the patient is also used.

### X-ray imaging system

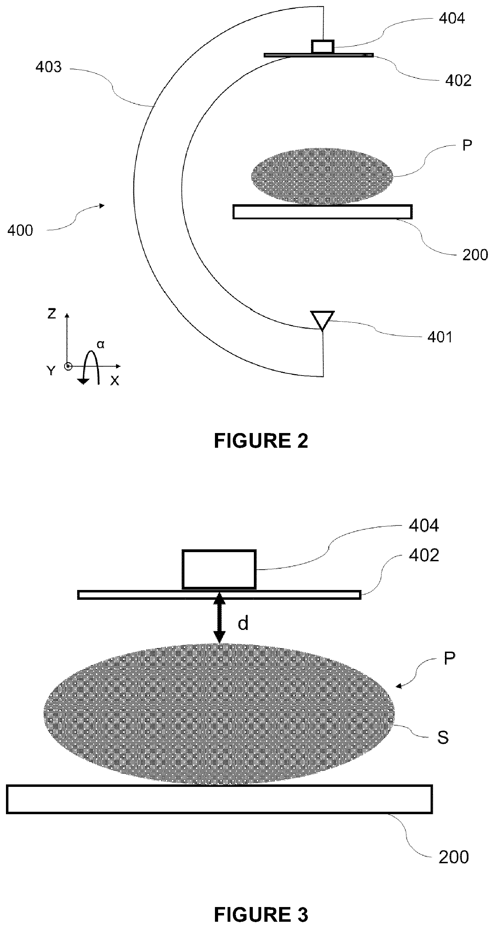

As depicted in more detail FIG. 2, the X-ray imaging system 400 comprises at least one X-ray source 401 and at least one X-ray image detector 402. The X-ray image detector may comprise a flat detector panel.

The X-ray source and X-ray image detector are carried by a C-shaped gantry 403, the X-ray source and X-ray image detector being arranged on opposite ends of the gantry. Due to the shape of the gantry, such an imaging system is usually called a C-arm. The center of the segment connecting the center of the X-ray source and the center of the detector is called the isocenter of the C-arm.

In a manner known *per se,* the X-ray imaging system is configured to produce at least one 2D X-ray image that is the result of a conical projection of a patient anatomy, wherein the tip of the cone is approximately the central point of the X-ray source and the basis of the cone is approximately the portion of the X-ray image detector that is reached by X-ray beams that have been collimated in a given shape and orientation. For example, the X-ray imaging system may be a conventional C-arm, or any Cone-Beam Computed Tomography (CBCT) imaging system such as the SURGIVISIO device (ECENTIAL ROBOTICS, Gieres, France), or VISION FD VARIO 3D (ZIEHM), CIOS SPIN MOBILE 3D (SIEMENS), AIRO (STRYKER), LOOP-X (BRAINLAB), O-ARM (MEDTRONIC).

A conventional C-arm is designed to allow the gantry to move relative to a base so as to move the X-ray source and detector about a patient while obtaining projection images of the patient placed between the X-ray source and the X-ray detector of the gantry.

In preferred embodiments, the X-ray imaging system may be motorized. In particular, the C-shaped gantry may comprise motors (not shown) allowing movement horizontally (X and Y directions), vertically (Z direction) and around the X direction (defined by an angle a), so that 2D X-ray images of the patient may be produced from almost any angle. As shown on FIG. 2, the X axis is transversal to the operating table 200 on which the patient lies and the Y axis is parallel to the longitudinal axis of said operating table.

Each motor is associated to an encoder that provides at any time the relative position of the X-ray imaging system with respect to a reference position. When a 2D X-ray image is acquired, the corresponding position of the imaging system is recorded. Thus, each 2D image is recorded in the referential of the imaging system.

The trajectory of the motorized C-arm is determined by each 2D X-ray image position of acquisition of said C-arm while performing a 3D image acquisition.

In some embodiments, the C-arm may comprise a mobile base 405 allowing displacing the C-arm in the operating room. The C-shaped gantry may thus be slidably and/or pivotably mounted on said mobile base. The motors of the C-arm may be arranged in the mobile base and/or in the gantry.

The C-arm is controlled by a control unit 406 which typically comprises a processor, a data storage device and a communication device.

The control unit may advantageously be embedded in the mobile base 405 of the X-ray imaging system. Said base may also comprise switches, such as a power switch, an emergency button and the like.

Alternatively, said control unit may be embedded in a separate cart (not illustrated) with at least one interface with the C-arm, or may be remote, for example in a separate control room of the hospital or in a data center.

Advantageously, the control unit of the robotic system and the control unit of the X-ray imaging system belong to a unified control system. By unified control system is meant in the present text that one of said control units runs a master computer-readable program and that the other control unit runs a slave computer-readable program, the master program being configured to send commands to the slave program and to receive data (e.g. status) from the slave program. For example, the control unit of the robotic system may run a master program to control movements of the robotic arm, while the control unit of the X-ray imaging system runs a slave program to control movements of the motorized C-arm and/or acquire X-ray images. Conversely, the control unit of the X-ray imaging system may run a master program to control movements of the motorized C-arm and/or acquire X-ray images, while the control unit of the robotic surgical system runs a slave program. In other embodiments, the master program could be run by a control unit of another system and the control units of the robotic system and the X-ray imaging system would both run slave programs. Thanks to such a unified control system, it is possible to synchronize actions of the robotic system and of the X-ray imaging system, since each system has knowledge of the position and status of the other system.

The unified control system advantageously comprises a user interface adapted to display information to the user.

### Anti-collision device

The X-ray imaging system further comprises an anti-collision device 404 fixed to a movable part of the C-arm. Said anti-collision device may be activated to detect obstacles in the vicinity of said movable part of the C-arm when it is moving. Such an anti-collision device may typically be used in a preliminary path of the C-arm, before launching acquisition of a set of 2D X-ray images, in order to make sure that said path does not involve any collision of the C-arm with the patient or any other object in the environment of the patient, such as the operating table. The anti-collision device may also be activated during acquisition of a set of 2D X-ray images, in order to prevent any collision of the C-arm with the patient or any other object in the environment of the patient, in particular if the environment has changed since the preliminary path.

The anti-collision device may rely on various sensor technologies to detect an obstacle in the vicinity of the C-arm. For example, but non-limitatively, the anti-collision device may comprise a proximity sensor (e.g. a capacitive sensor), a telemeter, a LIDAR (Laser Imaging, Detection And Ranging), a stereo camera, an ultrasound detector, a time-of-flight camera and/or a tactile sensor.

The anti-collision device is coupled to the control unit of the C-arm, so as to send to the control unit data regarding potential obstacles, such as a distance between the anti-collision device and an object.

The control unit may be configured to stop the movement of the C-arm in case the anti-collision device has detected an object at a distance smaller than a predetermined distance from the moving part of the C-arm.

In the method according to the present disclosure, the anti-collision device is used not only to detect obstacles and prevent a collision with such obstacles, as provided by the conventional use of such an anti-collision device, but also (or alternatively) to detect an outer surface of the patient's body.

As shown in FIG. 3, which is an enlarged view of the C-arm of FIG. 2, the anti-collision device 404 is capable of measuring a distance d between the outer surface S of the patient's body and the sensor(s) of the anti-collision device 404.

In order to increase the accuracy of the determination of the distance d between the outer surface of the patient's body and the anti-collision device 404, it may be advantageous to bring the anti-collision device 404 as close as possible to the patient.

Since the X-ray detector 402 has to be placed as close as possible to the patient to increase the size of the 3D reconstructed image, the anti-collision device 404 may, in preferred embodiments, be located on the X-ray detector 402, for example fixed to the side of the X-ray detector opposite to the detector panel, as shown in FIGS. 2 and 3. Alternatively, the anti-collision device may be attached to the gantry 403, preferably in the vicinity of the X-ray detector 402.

During a path of the C-arm, the anti-collision device 404 may thus send volumetric data of the patient's body to the control unit. Said data may be raw sensor data, for example in the form of an electric signal, a video signal or any other type of signal depending on the anti-collision technology, issued by the anti-collision device 404. The control unit then treats the raw sensor data to compute volumetric data of the patient's body. Alternatively, the anti-collision device 404 may itself send volumetric data to the control unit.

In practice, it is generally not necessary to obtain volumetric data of the whole patient's body, but only of a portion of the patient's body enclosing a region of interest which is to be imaged by the C-arm. For example, if the region of interest is a vertebra, the portion of the patient's body may be a portion of the patient's torso enclosing said vertebra and, if appropriate, adjacent vertebrae.

The volumetric data received or computed by the control unit may be typically a set of distances between the patient's body and the anti-collision device along the path of the motorized C-arm.

The path of the C-arm may be chosen to rotate around said portion of the patient's body in order to obtain a full set of volumetric data regarding the body portion. Alternatively, it may be possible to infer volumetric data from a partial set of data, regarding only a part of said body portion. For example, as the patient's body is considered to be symmetrical relative to the sagittal plane, it may be sufficient to obtain volumetric data for the body portion on one side of said sagittal plane. Besides, or alternatively, since the patient lies on the operating table, the surface of the body portion in contact with the operating table may be considered to coincide with the surface of the operating table - which may be detected by the anti-collision device - and it may thus be sufficient to obtain volumetric data for the body portion above the operating table.

Based on said volumetric data, the control unit of the X-ray imaging system is configured to compute a volumetric model of the patient's body portion. Thanks to the unified control system described above, said volumetric model can be transferred to the control unit of the robotic system for computing a prohibited volume.

### Prohibited volume

The prohibited volume may be defined as a translation, in the space of the robotic system, of the volumetric data generated (in the X-ray imaging system space) by the anti-collision system of the X-ray imaging system. Such a translation is particularly easy thanks to the coupling of the control units of the robotic system and the X-ray imaging system into a unified control system.

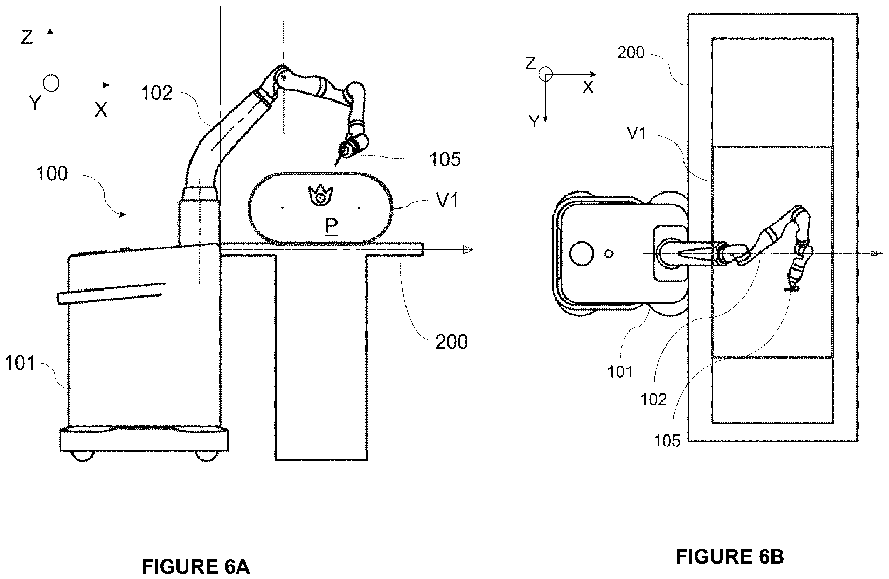

FIGS. 6A to 10B schematically illustrate side and top views of various non-limitative embodiments of the prohibited volume.

According to an embodiment illustrated in FIGS. 6A and 6B, the prohibited volume V1 may be strictly identical to the volume defined by the volumetric data, which is typically a portion of the patient's body. In particular, the dimensions of the prohibited volume in the X and Z directions are identical to the dimensions of the patient's body. In the Y direction, the prohibited volume does not necessarily have the same length as the patient's body, since the robotic arm may not be able to reach parts of the patient's body that are far from the region of interest and/or these parts are less prone to severe injuries.

Alternatively, the prohibited volume may be defined as the volume defined by a predetermined box whose dimensions in the X, Y plane substantially correspond to the dimensions of the operating table 200 (or of a part of the operating table) and whose superior edge contains the highest point (along the vertical axis Z) of the volume defined by the volumetric data. For example, the prohibited volume can be shaped as a rectangular box or an ellipsoid volume.

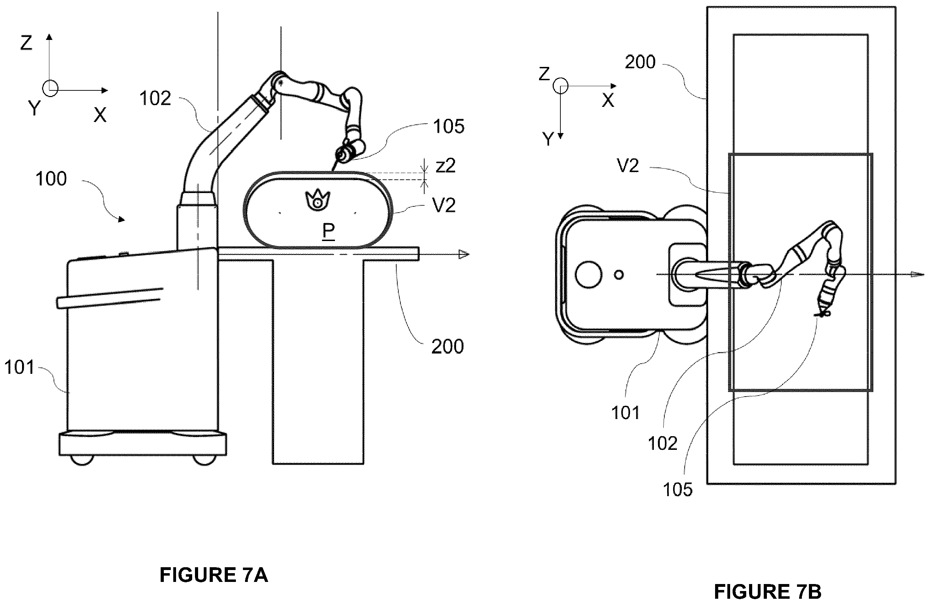

In some embodiments, as shown in FIGS. 7A and 7B, the prohibited volume V2 may be defined as the volume defined by the volumetric data with increased dimensions (substantially in height in the vertical direction Z) for taking into account breathing movements of the patient. Said additional height z2 can be of the order of a few centimeters.

More generally, the prohibited volume may be defined as the smallest volume containing the initial volume defined by the volumetric data and respecting specific criteria of shape (e.g. rectangular box, ellipsoid, etc.) and position (e.g. centered on the surgical target(s), centered on the patient, etc.).

In some embodiments, the prohibited volume may be divided into at least two sub-volumes that differ in terms of interaction with the surgical robotic system: for example, there may be several levels of counteraction by said robotic system, one level of counteraction corresponding to one sub-volume of the prohibited volume.

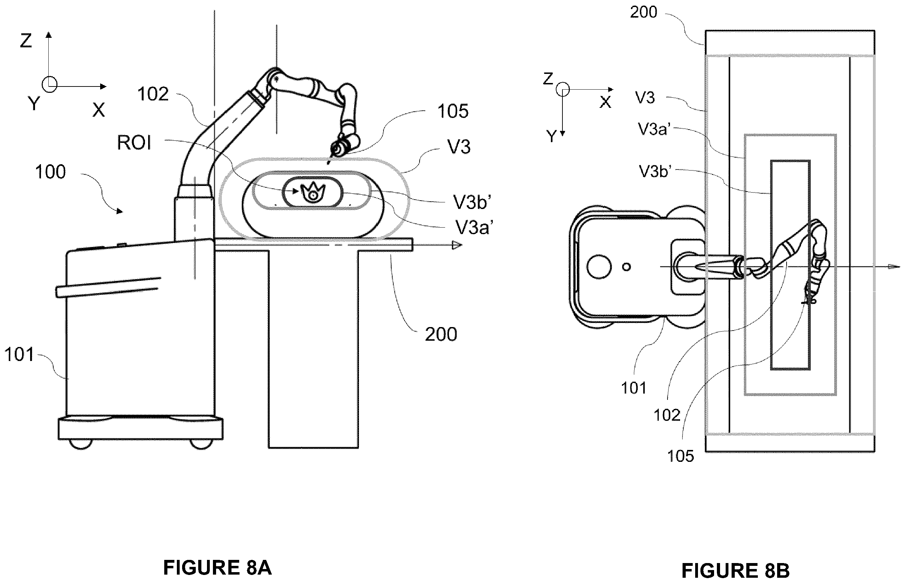

Said sub-volumes may be defined geometrically relative to the prohibited volume, or alternatively they may be defined anatomically. An illustration of the latter in FIGS. 8A-8B may be a sub-volume substantially corresponding to the patient's spine, including all vertebrae, spinal cord and nerve entry point in said spinal cord (together defined as a region of interest ROI), said sub-volume V3a' corresponding to drastic counteraction of the surgical robotic system in case of trespassing of any part of said system into said sub-volume. Such drastic response may be necessary due to the risk of permanent damages to vulnerable and vital parts of the patient. Furthermore, another sub-volume V3b' corresponding to the sides of the patient may be defined, said sub-volume corresponding to more permissive counteraction of the surgical robotic system in case of trespassing of any part of said system into said sub-volume. The sub-volume V3b' encloses the sub-volume V3a', both sub-volumes being enclosed in the prohibited volume V3 which extends beyond the surface of the patient's body in X and Z directions to include a safety zone.

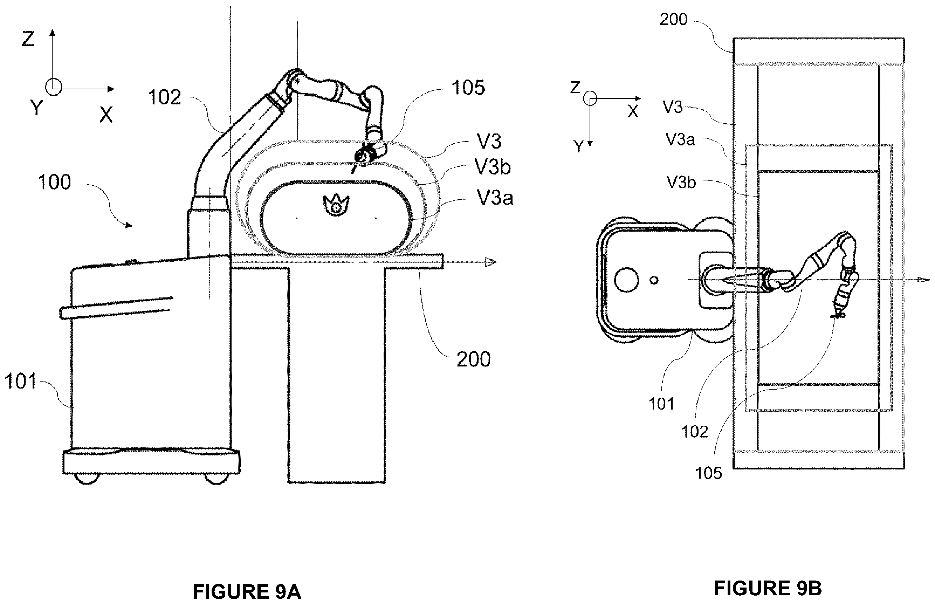

Another embodiment, illustrated in FIGS. 9A-9B, may be the definition of a first sub-volume V3a based on the volumetric data collected by the anti-collision device, i.e. the volume corresponding to the patient, and the definition of at least one second sub-volume V3b, V3 with more permissive counteraction corresponding to the area above the patient, for example an area of a few centimeters above the patient.

In some embodiments, the prohibited volume may additionally take into account the information input by the surgeon during the planning of the surgical procedure on the 2D and/or 3D images acquired preoperatively.

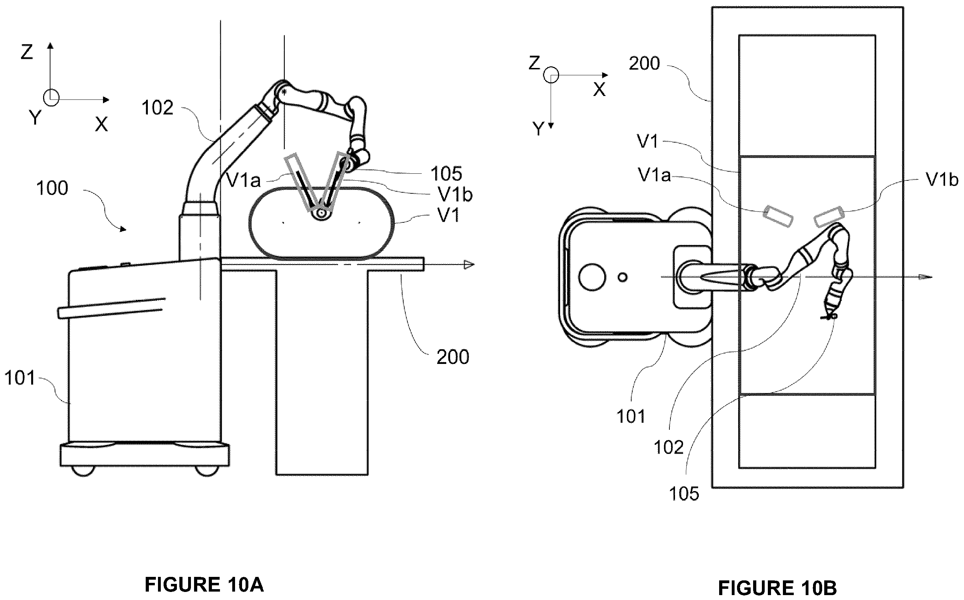

For example, as shown in FIGS. 10A-10B, in the case of pedicle screw placement, the surgeon may indicate a drilling axis that the robotic arm will have to maintain for the drill bit through the positioning of each pedicle screw. As the robotic arm and the tool held or guided by the robotic arm may have to evolve at least partially near these axes during drilling, it may be advantageous to exclude volumes Via, V1b around said axes from the prohibited volume V1. Said volumes V1a, V1b may be cylinders or cones whose axis of revolution correspond to the planned drilling axis.

### Method for defining the prohibited volume

During a surgical intervention, the patient P lies on the operating table 200.

For example, in case of spine surgery, the patient may lie face down on the operating table, such that the region of interest, which may comprise one or several vertebrae, is accessible to the surgeon.

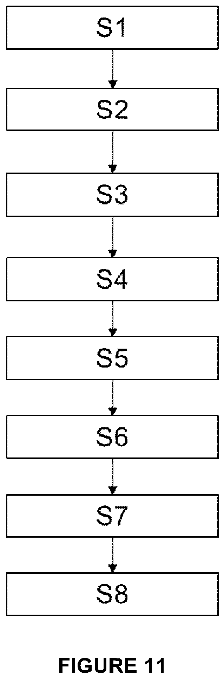

FIG. 11 is a flowchart illustrating how the unified control system can be operated to define the prohibited volume and control the robotic surgical system accordingly. This flowchart is only a non-limitative example and some steps may be omitted or carried out in a different order if appropriate.

In an initial step S1, the master program of the unified control system sends an initialization sequence command to all slave programs. As mentioned above, the master program may be run by the control unit of the X-ray imaging system, by the control unit of the robotic surgical system or by a control unit of another system coupled to the X-ray imaging system and the robotic system. Each one of the control unit of the X-ray imaging system and the control unit of the robotic surgical system runs at least one slave program dedicated to a respective functionality of the system.

The slave program in charge of the movements of the motorized C-arm places the C-arm in a default position for the beginning of surgery.

The slave program in charge of the shot parameters of the motorized C-arm configures the X-ray source and associated subsystems (collimator, filters) in a default state (for example, the slave program sets default kV/mA value, pulse size and frequency, filter configuration, default collimator configuration...).

In step S2, a path of the C-arm is computed by a slave program so as to acquire a set of 2D X-ray images of the region of interest and reconstruct a 3D medical image based on said set of 2D X-ray images. The trajectory of the C-arm may involve at least one rotation, but it may also be more complex and combine at least one rotation and one translation, or several rotations. In some embodiments, the trajectory of the C-arm may be computed to increase the size of the reconstructed X-ray image or minimize patient's irradiation, which requires bringing the X-ray detector as close as possible to the patient, without colliding with the patient or any object in the vicinity of the patient, such as the operating table.

The master program receives the computed path and sends the actuation commands corresponding to the successive positions of said computed path to a slave program in charge of the movements of the motorized C-arm. In a similar fashion, the master program sends for each said acquisition position the shot parameters of the X-ray imaging system to the slave program in charge of the shot parameters.

Then, the master program waits for the acquisition data (i.e. all 2D X-ray images acquired during the acquisition path) sent by the slave program in charge of the acquisition of the X-ray images and the volumetric data sent by the slave program in charge of the anti-collision device.

In some embodiments, a first path of the C-arm may be implemented around a portion of the patient's body including the region of interest, without acquiring any 2D X-ray image. The first path comprises a plurality of positions of the X-ray source and X-ray detector relative to the patient about at least one rotation axis, and, if appropriate about additional rotation or translation axes.

Along said first path, the anti-collision device is activated.

For each position of the C-arm along the first path, the anti-collision device detects not only the presence of objects in the environment of the C-arm, but also the external surface of the patient's body.

As a result, a set of volumetric data relating to the portion of the patient's body is recorded in a memory of the unified control system. Said volumetric data may comprise a distance between the X-ray image detector and the patient's body portion for each angular position of the C-arm.

After the first path has been completed, a second path of the C-arm can be implemented around the same portion of the patient's body to acquire a set of 2D X-ray images at each angular position of the C-arm.

In some embodiments, the second path is identical to the first path. Alternatively, the second path may be different from the first path. For example, the anti-collision device may have detected an obstacle during the first path, and the second path has been adjusted to avoid said obstacle. In other situations, the first path may have been implemented without minimizing the distance between the X-ray detector and the patient's body, and the second path may be optimized to minimize said distance, as explained above.

In some embodiments, only one path of the C-arm is implemented to acquire 2D X-ray images, with the anti-collision device activated. Otherwise said, there is no preliminary path to detect potential collisions, but since the anti-collision device is activated, it allows preventing collisions of the C-arm with the patient or other object in the vicinity of the C-arm.

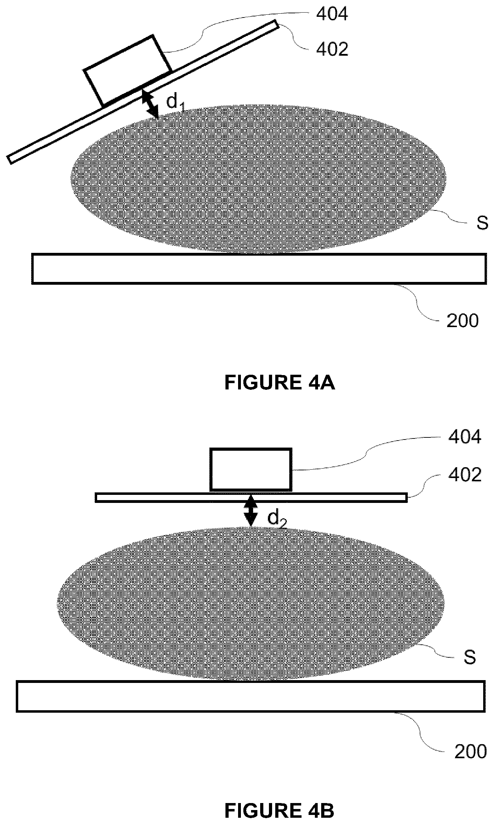

FIGS. 4A and 4B schematically illustrate two different positions of the X-ray detector relative to the patient's body along a path of the motorized C-arm, in which the X-ray detector 402 is at a distance d1, d2, respectively, from the outer surface S of the patient's body, measured by the anti-collision device 404.

In step S3, when the first path has been completed, the master program sends a command to a slave program to compute a volumetric model of the portion of the patient's body based on the set of volumetric data.

The volumetric model is stored in a memory of the unified control system.

In some embodiments, the first path may be optimized to have the X-ray detector as close as possible to the patient's body.

To that end, the first path may be computed by, for each angular position of the C-arm of said trajectory, computing a translation of the C-arm along a central axis extending between the X-ray source and a center of the X-ray image detector and passing by the center of the region of interest to reduce a distance between the X-ray image detector and the center of the region of interest whilst avoiding collisions between the X-ray source and detector and the operating table and/or the body.

Such a path, if implemented during 2D X-ray image acquisition, allows maximizing the size of the 3D image and minimizing the X-ray dose received by the patient.

Indeed, each 2D image corresponds to a conical projection of the region of interest onto the X-ray detector. The size of the reconstructed 3D volume is related to the size of the intersection of every cone of projection of a 3D acquisition. Thus, as the C-arm trajectory is such that each cone of projection meets the region of interest at its largest, i.e. if the detector is as close as possible of said region of interest, the result will be an optimal reconstructed volume in terms of size. In addition, since the region of interest is farther from the X-ray source, the X-ray dose received by the patient is reduced.

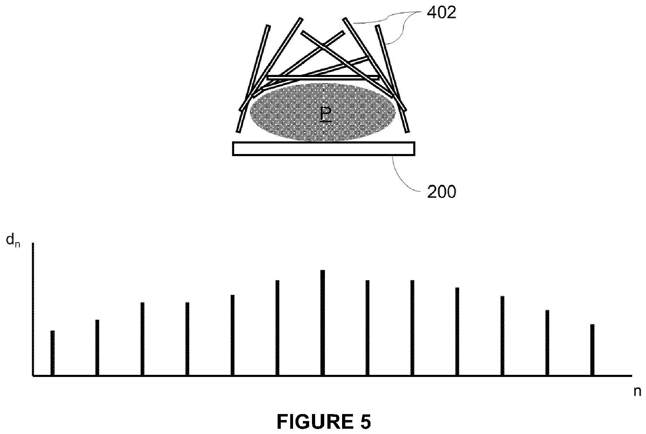

FIG. 5 (top) illustrates a plurality of positions of the X-ray detector 402 with respect to the patient's body P along such an optimized first path. In each of said positions, the X-ray detector is as close as possible to the patient's body, while avoiding any collision between the C-arm and the operating table 200 or the patient's body P.
FIG. 5 (bottom) illustrates a graph presenting the distance dₙ between the X-ray detector and the outside surface of the patient's body at each respective position n.

Said distances, associated with the respective angular position of the C-arm, form the set of volumetric data that is used to compute the volumetric model.

The C-arm being motorized, each motor is associated to an encoder that provides at any time the relative position of the X-ray imaging system with respect to a reference position. As a result, even if the second path is different from the first path, the unified control system is always able to establish a spatial relationship between a position of the C-arm during acquisition of a 2D X-ray image and the volumetric model.

The acquired set of 2D X-ray image is used to reconstruct the 3D image.

To that end, in step S4, the master program sends an action command to a slave program in charge of the reconstruction of the 3D image to reconstruct the volume of the region of interest from all the 2D X-ray images of the region of interest acquired at the previous step and sent back by the slave program in charge of the acquisition of the X-ray images.

Optionally, the master program may send an action command to a slave program in charge of the user interface to display the reconstructed 3D image for the surgeon.

In the unified control system, the translation of the volumetric model into the prohibited volume can be implemented as follows.

As mentioned above, a slave program computes, based on the volumetric data provided by the anti-collision device, the volumetric model of the patient's body portion. Since the C-arm is motorized, the position of the C-arm for each acquisition of the volumetric data and for each acquisition of a 2D X-ray image is known.

During acquisition of the 2D X-ray images, a registration phantom is rigidly attached to the patient. The registration phantom comprises radiopaque fiducials that are detectable in the 2D X-ray images. Advantageously, as described in particular in document WO 2017/064287, the registration phantom can be removably attached to a base attached to an anatomical structure of the patient, the base being also configured to removably receive a tracker detectable by the localization system in order to navigate the surgical tool during the surgical procedure. The registration phantom and the tracker sharing a same base, the position of the 2D X-ray images in the coordinate system of the localization system can be determined.

Alternatively, a tracker could be attached to the movable part of the C-arm in order to be tracked by the localization system, but such a solution is less preferred since it is more constraining. In particular, one has to ensure that the tracker is detectable by the localization system in any position of the C-arm, which is difficult in view of the large range of motion of the C-arm.

In addition, since a tracker is attached to the end-effector and tracked by the localization system, the position of the robotic arm and the end-effector can be determined relative to the 2D X-ray images and thus relative to the volumetric model of the patient's body.

In step S5, a slave program computes the prohibited volume based on the volumetric model and, if appropriate, on additional constraints, such as the shape of the prohibited volume, the inclusion of a safety zone to take into account breathing movements, or the provision of several sub-volumes, as described above.

The user interface of the unified control system advantageously allows a user to visualize the prohibited volume and, if appropriate, modify the prohibited volume.

To that end, in step S6, the master program sends a command to the slave program in charge of the user interface to display the prohibited volume and to receive modifications from the user.

Said modifications may relate in particular to dimensions or position of the prohibited volume, or to level of counteraction of the robotic arm.

Once the prohibited volume has been defined and, if appropriate, validated or modified, it is used by the control unit of the robotic system to control movements of the robotic arm and end-effector.

### Operation of the robotic surgical system

The surgical intervention is planned by the user to define at least one surgical target. Each surgical target may include in particular an entry point, a target axis and/or a target plane.

In step S7, the slave program in charge of the user interface receives the planning information given by the user through said interface and sends it to the master program.

In step S8, during implementation of the surgical procedure, the master program sends an action command to the slave program in charge of the movements of the robotic arm of the surgical robotic system to align the tool guide or the tool held by the end-effector with each planned target.

The slave program in charge of the movements of the robotic arm periodically sends information on its position to the master program.

The master program thus determines a position of the robotic arm and end-effector relative to the prohibited volume, and/or a speed of displacement of the robotic arm and end-effector toward the prohibited volume.

If a part of the robotic arm or the end-effector is going towards the prohibited volume, the master program may send a command to a slave program to generate an optical, sound-based or haptic signal to warn the user of a potential risk of entering the prohibited volume. Said signal may vary depending on the speed of displacement of the robotic arm or end-effector, or depending on the distance between the robotic arm or end-effector and the prohibited volume.

In addition or alternatively, when a part of the surgical robotic system is about to enter said volume, the master program may send a command to a slave program to activate at least one counteraction, for example by generating a signal to immediately stop any movements of any parts of the surgical robotic system, to move a part of the surgical robotic system in an opposite direction relative to the prohibited volume, and/or to reduce the speed of at least one motor of the robotic arm.

At last, the user can also stop the robotic surgical system using an emergency stop button.

## Claims

1. Method for determining a prohibited volume for a surgical robotic system comprising a control unit, a robotic arm (102) and an end-effector (105) adapted to treat a region of interest of a patient's body, comprising:
- implementing a first path of a motorized C-arm (403) supporting an X-ray source (401), an X-ray image detector (402) and an anti-collision device (404), said first path comprising at least two different angular positions around a rotation axis of the C-arm;
- at each angular position of the C-arm along said first path, detecting with the anti-collision device an external surface of the patient's body and recording volumetric data of a patient's body portion enclosing the region of interest;
- computing a volumetric model of the patient's body portion based on said volumetric data;
- sending the volumetric model to the control unit of the surgical robotic system,
- based on said volumetric model, defining a prohibited volume (V1, V2, V3) for the robotic arm and end-effector.

2. Method according to claim 1, wherein the prohibited volume (V1) is identical to the volume of the volumetric model of the patient's body portion.

3. Method according to claim 1, wherein the prohibited volume (V2) is computed to enclose the volumetric model of the patient's body portion.

4. Method according to claim 3, wherein the prohibited volume (V2) is computed to include a safety zone extending outside the volumetric model of the patient's body portion.

5. Method according to claim 3, wherein the prohibited volume (V3) comprises first and second sub-volumes each enclosing the volumetric model of the patient's body portion, the first sub-volume (V3a) being enclosed in the second sub-volume (V3b).

6. Method according to claim 1, wherein the prohibited volume (V3) comprises a first sub-volume (V3a) enclosing a region (ROI) of the volumetric model and at least one second sub-volume (V3b) enclosing the first sub-volume (V3a).

7. Method according to any one of claims 1 to 6, comprising receiving at least one planned target axis or plane, computing an authorized volume (V1a, V1b) around said planned target axis or plane and excluding said authorized volume (V1a, V1b) from the prohibited volume (V1).

8. Method according to any one of claims 1 to 7, further comprising generating a warning signal if the robotic arm (102) and/or the end effector (105) moves toward the prohibited volume.

9. Method according to any one of claims 1 to 7, further comprising generating a warning signal if the robotic arm (102) and/or the end effector (105) is at a distance from the prohibited volume smaller than a predetermined distance.

10. Method according to any one of claims 1 to 9, wherein the anti-collision device (404) comprises at least one of: a proximity sensor, a telemeter, a LIDAR, a stereo camera, an ultrasound detector, a time-of-flight camera and a tactile sensor.
